# EUROPÄISCHE PATENTSCHRIFT

(11) **EP 0 769 271 B1**
(45) Veröffentlichungstag und Bekanntmachung des Hinweises auf die Patenterteilung: **03.12.2003**
(21) Anmeldenummer: 96116808.5
(22) Anmeldetag: 18.10.1996
(51) Int. Cl.: A61B 17/02, A61F 9/00

(54) **Vorrichtung zum Spreizen einer Augeniris**
Device for expanding the iris of an eye
Dispositif pour écarter l'iris d'un oeil

(30) Priorität: 19.10.1995 DE 19538951
(43) Veröffentlichungstag der Anmeldung: 23.04.1997
(73) Patentinhaber: Holzwig, Detlef H., Dr., 40213 Düsseldorf (DE)
(72) Erfinder: Holzwig, Detlef H., Dr., 40213 Düsseldorf (DE)
(74) Vertreter: Nöth, Heinz, Dipl.-Phys.

(56) Entgegenhaltungen:
- WO-A-93/14703
- US-A- 4 782 820
- US-A- 5 163 419
- US-A- 5 267 553
- US-A- 5 374 272

## Beschreibung

Die Erfindung betrifft eine Vorrichtung zum Spreizen einer Augeniris.

Bei Patienten, welche eine enge Pupille, d.h. eine enge Irisöffnung, haben, sind Operationen im Bereich der Hinterkammer des Auges schwierig und mit hohem Verletzungsrisiko für die Iris verbunden. An der engen Pupille kann sogar das Gelingen der gesamten Operation scheitern.

Aus dem US-Patent 5,163,419 geht eine Vorrichtung der eingangs genannten Art hervor, deren Spreizkörper zwei Schenkel aufweist, die über einen gerade, im wesentlichen quer zu diesen Schenkeln verlaufenden Steg miteinander verbunden sind. Die freien Enden der Schenkel sind jeweils mit einer Verdickung versehen, die an ihren Außenseiten ebenso wie der die beiden Schenkel miteinander verbindende Steg mit einer Nut versehen sind, in die die Ränder der Iris eingreifen, nachdem die Vorrichtung platziert worden ist. Bei dieser bekannten Vorrichtung sind nur geringe Abschnitte des Irisrandes aufgrund des geraden Steges und der Verdickungen in Eingriff mit den vorgesehenen Nuten gebracht. Aufgrund des geraden im wesentlichen quer zu den Schenkeln verlaufenden Steges ist für das Einsetzen des Spreizkörpers in das Auge ein relativ breiter Schnitt erforderlich.

Aus der US-A-5,374,272 ist eine Vorrichtung zum Spreizen einer Augeniris bekannt, deren Spreizkörper ringförmig ausgebildet ist und am Außenumfang eine umlaufende Nut für einen Eingriff am Innenrand der Irisrand aufweist. Um den ringförmigen Spreizkörper in das Auge zu implantieren, ist ein relativ breiter Schnitt am Auge zu legen.

Aus der US-A-5,267,553 ist eine Vorrichtung zum Spreizen einer Augeniris bekannt, bei welcher der Spreizkörper ebenfalls im wesentlichen ringförmig mit einer umlaufenden Eingriffsnut an der Außenseite ausgebildet ist. Um diesen ringförmigen Spreizkörper beim Implantieren in eine langgestreckte Form zu bringen, sind relativ aufwendige Maßnahmen erforderlich.

Bei der aus der WO-A-93/14703 bekannten Vorrichtung zum Spreizen der Iris wird der Spreizkörper in dehydradisierter Form in das Auge eingesetzt und durch Hydradisierung in die ringförmige expandierte Form gebracht.

Aufgabe der Erfindung ist es daher, eine Vorrichtung zum Spreizen einer Augeniris zu schaffen, welche einfach implantierbar ist.

Diese Aufgabe wird erfindungsgemäß durch die Merkmale des Anspruchs 1 gelöst.

Hierdurch wird die Gefahr einer Miosis (Engstellung) der Pupille während eines operativen Eingriffes in die Hinterkammer durch die Pupillenöffnung hindurch, beispielsweise während einer Kataraktoperation, verhindert.

Der in V-Form ausgebildete Spreizkörper, welcher beispielsweise aus Silikongummi bestehen kann und somit zu einer verengten Form sich verformen läßt, kann in einfacher Weise durch einen Tunnel mit insbesondere bogenförmigem Schnitt im Bereich der Sclera etwa in einem Abstand von 2 mm zum Limbus oder durch einen Hornhaut-Tunnel (clear comea-Technik) in die Öffnung der Iris eingesetzt werden. Ein geeignetes Material für den Spreizkörper ist auch Polymethylmethacrylat oder dergl.

Für die Fixierung des Spreizlörpers und für den Eingriff in den Innenrand der Iris ist an seinem Umfang ein an den Innenrand der Iris angepaßtes Profil vorgesehen. In bevorzugter Weise handelt es sich hier um ein an den äußeren Flächen der beiden Schenkel des V-förmigen Spreizkörpers vorgesehenes Profil. Dieses Profil kann einen V-förmigen Querschnitt aufweisen, das nach außen geöffnet ist.

Bei der Implantation des V-förmigen Spreizkörpers wird der Innenrand der Iris in dieses V-förmige Profil eingesetzt. Durch die insbesondere V-Form des Profils wird eine einwandfreie Fixierung des Spreizkörpers, welcher als Irisretraktor wirkt, am Innenrand der Augeniris erzielt.

Wenn die Operation, insbesondere Kataraktoperation, mit Tunneltechnik durchgeführt wird, kann der in der Sclera angelegte Tunnel oder der in der Hornhaut (clear cornea) Tunnel-Schnitt auch für die Implantation des Spreizringes (Irisretraktor) verwendet werden. Hierzu wird der V-förmige Spreizkörper zu einer verengten Form mit etwa parallelen Schenkeln mit einer Breite von geringer als 3 mm mit Hilfe eines Werkzeugs, beispielsweise einer Pinzette, zusammengedrückt und durch die Tunnelöffnung in die Pupille eingesetzt. Anschließend wird die Operation durchgeführt. Nach Beendigung der Operation erfolgt das Entfemen des Spreizkörpers (Retraktors) aus der Pupille und Verschließen der Tunnelöffnung.

Anhand der Figuren wird an einem Ausführungsbeispiel die Erfindung noch näher erläutert. Es zeigt:

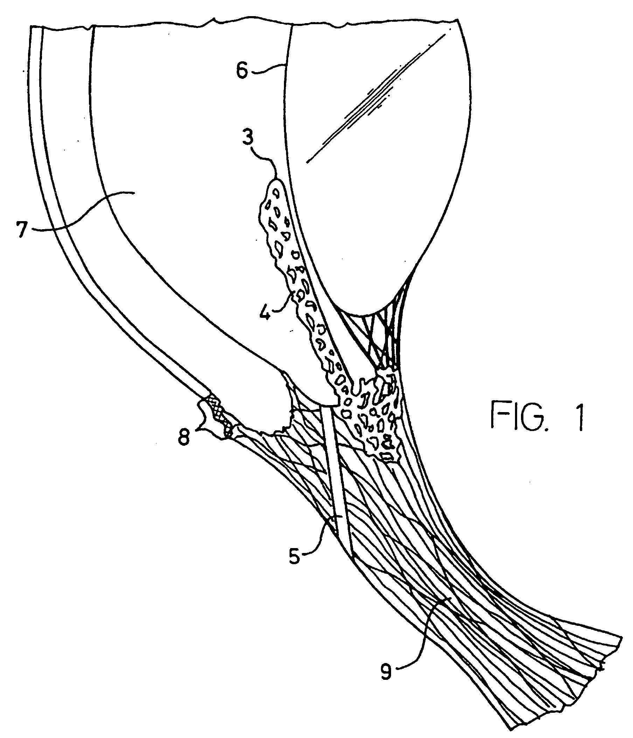

- Fig. 1:: einen Schnitt durch einen Teil eines Auges zur Erläuterung der Implantation des erfindungsgemäßen Spreizkörpers in die Augenpupille;
- Fig. 2(A):: in Draufsicht ein Ausführungsbeispiel eines erfindungsgemäßen Irisretraktors
- Fig. 2(B):: in perspektivischer Darstellung einen Teil dieses Ausführungsbeispiels; und
- Fig. 3.: eine Draufsicht auf das Auge zur Erläuterung der Implantation des in Fig. 2 dargestellten Ausführungsbeispiels.

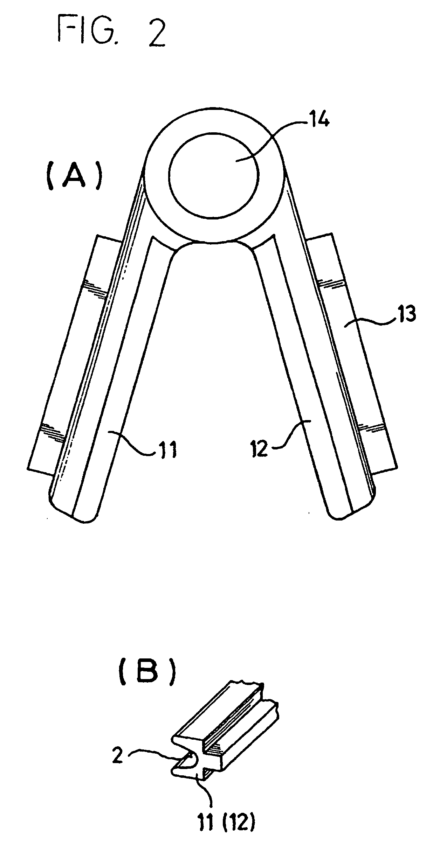

Bei dem in der Figur 2 dargestellten Ausführungsbeispiel eines Irisretraktors ist der Spreizkörper ausgebildet in V-Form. Der Spreizkörper besitzt zwei Schenkel 11 und 12. Die beiden Schenkel sind in der Spitze, in welcher sie im Winkel zusammenlaufen, über ein zylindrisches Verbindungsstück 14 miteinander verbunden. Das Material des dargestellten Ausführungsbeispiels kann Silikongummi, Polymethylmethacrylat oder auch Metall sein.

Die beiden Spreizschenkel 11 und 12 besitzen an ihren Außenflächen jeweils das Profil 2, welches in den Innenrand 3 der Iris 4 eingreift. Zusätzlich können schildförmige Flächenverbreiterungen 13 vorgesehen sein, welche einen zusätzlichen Schutz für die Iris bedeuten. Diese schildförmigen Verbreiterungen der Außenflächen der beiden Schenkel 11 und 12 können das Gesamtprofil C-förmig erweitern. In der Figur 2(A) ist eine Draufsicht auf das Ausführungsbeispiel und in der Figur 2(B) eine perspektivische Darstellung eines Stückes eines Schenkels dargestellt.

Der Profilboden kann, wie es gezeigt ist, abgerundet sein, wobei die Rundung einen Radius von etwa 0,2 mm hat. Die Tiefe des Profils beträgt beim Ausführungsbeispiel etwa 0,5 mm. Die Profilöffnung am Umfang kann 0,4 bis 0,6 mm betragen.

Die Dicke des Spreizkörpers beträgt beim Ausführungsbeispiel etwa 1 mm. Auch die Breite des Spreizkörpers beträgt etwa 1 mm.

Alle sterilen und sonstigen hygienischen Anforderungen der Augenheilkunde lassen sich bei dem erfindungsgemäßen Irisretraktor erfüllen.

Bei der Durchführung einer Kataraktoperation ergibt sich folgender Verfahrensablauf:

Nach den vorbereitenden Schritten, nämlich Spritzen von Beruhigungsmitteln, retrobulbärer Anästhesie und Fixation der Augenlider sowie Anschlingen des Muskulus rectus superior wird die Bindehaut am Limbus 8 eröffnet.

Im Bereich der Sclera 9 etwa in einem Abstand von 2 mm zum Limbus 8 wird für die Tunneltechnik ein bogenförmiger Schnitt gesetzt. Dies erfolgt in bekannter Weise mit einem Diamantmesser bzw. Tellermesser. Der Tunnel 5 wird zunächst so angelegt, daß die Vorderkammer 7 des Auges nicht eröffnet wird.

Anschließend wird mittels Kapsulorhexis die vordere Kapsel 6 geöffnet durch eine Parazenthese und mit einer Kanüle.

Dann wird mit einer etwa 3 mm breiten Lanze im Tunnelbereich die Vorderkammer des Auges eröffnet.

Nach Eröffnung der Vorderkammer 7 erfolgt das Einsetzen des Irisretraktors in die Pupille 10, so daß der Innenrand 3 der Iris im umlaufenden Profil 2 des Spreizkörpers eingesetzt ist. Hierdurch wird die Pupille in ihrem erweiterten Zustand gehalten, und es steht im Pupillenbereich eine Öffnung zur Verfügung mit dem gespreizten Abstand der Schenkel 11, 12.

Anschließend erfolgt das Zertrümmern und Absaugen des Kerns und der Rinde des natürlichen Linsenmaterials sowie Spülen und Absaugen von Linsenmaterialresten aus der Kapsel.

Anschließend wird eine Intraokutarlinse duch den Tunnel 5 in die geöffnete Kapsel des Auges eingesetzt. Bei Verwendung einer faltbaren Linse, welche beispielsweise mit Hilfe eines aus der deutschen Patentschrift DE 41 08 303 C2 bekannten Faltwerkzeuges gefaltet worden ist, kann die Linse ohne Erweiterung des Tunnelschnittes durchgeführt. werden. Für das Einsetzen der gefalteten Linse kann ein Implantationswerkzeug verwendet werden, wie es aus der deutschen Patentschrift 40 30 492 bekannt ist.
Nach Einsetzen der Linse wird der Irisretraktor aus der Pupille 10 wieder entfernt.

Die Vorderkammer 7 des Auges wird wieder aufgefüllt, insbesondere mit Kochsalzlösung. Die Bindehaut wird wieder vorgezogen und beispielsweise durch Koagulation mit dem übrigen Augengewebe verbunden.

Durch den Einsatz des erfindungsgemäßen Irisretraktors hat sich die Kataraktoperation ohne Verletzungsrisiko der Iris 4 durchführen lassen, wobei das Implantieren und Explantieren des Spreizkörpers durch den Tunnel 5 erfolgte, durch welchen auch die Implantation der Intraokularlinse erfolgte.

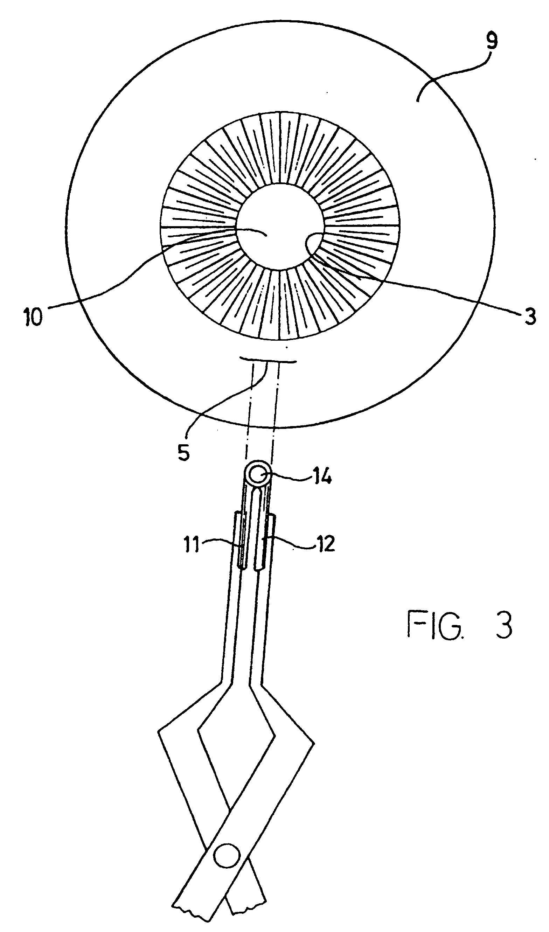

In der Figur 3 ist gezeigt, wie mit Hilfe einer Pinzette die beiden Schenkel 11 und 12 zusammengedrückt werden, so daß sie einen etwa parallelen Verlauf haben. Nach der Implantation werden die beiden Schenkel 11 und 12 durch die Spreizkraft auseinandergebracht, wodurch die Iris in Richtung auf den Kammerwinkel zurückgedrängt wird. Durch die gegebenenfalls vorgesehenen Schutzschilde 13 wird die Iris noch zusätzlich geschützt. Es ist noch möglich, an den Enden der beiden Schenkel 11 und 12 Ösen vorzusehen, um eine weitere Fixierung zu haben.

Im implantierten Zustand stützt sich der Retraktor ferner am äußeren Umfang des zylindrischen Verbindungsstückes 14 am Innenrand 3 der Iris ab. Das Profil 2 verläuft daher auch um den Außenumfang des zylindrischen Verbindungsstückes 14.

## Patentansprüche

1. Vorrichtung zum Spreizen einer Augeniris, mit einem Spreizkörper (11, 12, 14), der zwei miteinander verbundene Schenkel (11, 12) aufweist und der an seiner nach außen gerichteten Fläche ein Profil (2) für einen Eingriff am Innenrand der Iris (4) aufweist,
**dadurch gekennzeichnet, dass** die beiden Schenkel (11, 12) V-förmig miteinander verbunden sind und dass das Profil (2) als durchgehendes Profil jeweils an den äußeren Flächen der beiden Schenkel (11, 12) vorgesehen ist.

2. Vorrichtung nach Anspruch 1,
**dadurch gekennzeichnet, dass** das Profil (2) einen etwa V-förmigen Querschnitt aufweist, der nach außen geöffnet ist.

3. Vorrichtung nach Anspruch 1 oder 2,
**dadurch gekennzeichnet, dass** die Dicke und Breite des Spreizkörpers (11, 12, 14) etwa 1 mm betragen.

4. Vorrichtung nach einem der Ansprüche 1 bis 3,
**dadurch gekennzeichnet, dass** der Spreizkörper (11, 12, 14) aus Silikongummi besteht.

5. Vorrichtung nach einem der Ansprüche 1 bis 4,
**dadurch gekennzeichnet, dass** der Spreizkörper aus Polymethylmethacrylat (PMMA) besteht.

6. Vorrichtung nach einem der Ansprüche 1 bis 5,
**dadurch gekennzeichnet, dass** das Profil (2) eine Tiefe von ca. 0,5 mm hat.

7. Vorrichtung nach einem der Ansprüche 1 bis 6,
**dadurch gekennzeichnet, dass** das Profil (2) an seinem Boden abgerundet ist.

8. Vorrichtung nach Anspruch 7,
**dadurch gekennzeichnet, dass** der abgerundete Boden einen Radius von ca. 0,2 mm aufweist.

9. Vorrichtung nach einem der Ansprüche 1 bis 8,
**dadurch gekennzeichnet, dass** die Profilöffnung etwa 0,4 mm bis 0,6 mm beträgt.

10. Verwendung einer Vorrichtung nach einem der Ansprüche 1 bis 9, bei welcher der Spreizkörper (11, 12, 14) durch einen in der Sclera (9) oder Hornhaut des Auges angelegten und in die Augenvordervorkammer mündenden Tunnel (5) in die Pupillenöffnung der Iris (4) zum Spreizen der Augeniris eingesetzt wird.

## Claims

1. A device for retracting the iris of an eye, comprising a retractor body (11, 12, 14) which has two legs (11, 12) which are connected together and which at its outwardly directed surface has a profile (2) for engagement at the inner edge of the iris (4), **characterised in that** the two legs (11, 12) are connected together in a V-shape and that the profile (2) is provided in the form of a continuous profile at each of the outer surfaces of the two legs (11, 12).

2. A device according to claim 1 **characterised in that** the profile (2) is of a substantially V-shaped cross-section which is open outwardly.

3. A device according to claim 1 or claim 2 **characterised in that** the thickness and width of the retractor body (11, 12, 14) are about 1 mm.

4. A device according to one of claims 1 to 3 **characterised in that** the retractor body (11, 12, 14) comprises silicone rubber.

5. A device according to one of claims 1 to 4 **characterised in that** the retractor body (11, 12, 14) comprises polymethylmethacrylate (PMMA).

6. A device according to one of claims 1 to 5 **characterised in that** the profile (2) is of a depth of about 0.5 mm.

7. A device according to one of claims 1 to 6 **characterised in that** the profile (2) is rounded off at its bottom.

8. A device according to claim 7 **characterised in that** the rounded-off bottom is of a radius of about 0.2 mm.

9. A device according to one of claims 1 to 8 **characterised in that** the opening of the profile is about 0.4 mm to 0.6 mm.

10. Use of a device according to one of claims 1 to 9 in which the retractor body (11, 12, 14) is inserted into the pupil opening of the iris (4) to retract the iris of the eye through a tunnel (5) provided in the sclera (9) or cornea of the eye and which opens into the anterior chamber of the eye.

## Revendications

1. Dispositif pour écarter l'iris d'un oeil, comprenant un écarteur (11, 12, 14) qui comporte deux branches reliées l'une à l'autre (11, 12) et qui comporte sur sa surface dirigée vers l'extérieur un profil (2) pour un contact sur le bord interne de l'iris (4),
**caractérisé en ce que** les deux branches (11, 12) sont reliées l' une à l'autre en forme de V et que le profil (2) est prévu en tant que profil continu sur la surface externe de chacune des deux branches (11, 12).

2. Dispositif selon la revendication 1,
**caractérisé en ce que** le profil (2) comporte une section transversale à peu près en forme de V qui est ouverte vers l'extérieur.

3. Dispositif selon la revendication 1 ou 2,
**caractérisé en ce que** l'épaisseur et la largeur de l'écarteur (11, 12, 14) sont d'environ 1 mm.

4. Dispositif selon l'une des revendications 1 à 3,
**caractérisé en ce que** l'écarteur (11, 12, 14) est en caoutchouc de silicone.

5. Dispositif selon l'une des revendications 1 à 4,
**caractérisé en ce que** l'écarteur est en polyméthacrylate de méthyle (PMMA).

6. Dispositif selon l'une des revendications 1 à 5,
**caractérisé en ce que** le profil (2) a une profondeur d'environ 0, 5 mm.

7. Dispositif selon l'une des revendications 1 à 6, **caractérisé en ce que** le profil (2) a un fond arrondi.

8. Dispositif selon la revendication 7,
**caractérisé en ce que** le fond arrondi comporte un rayon d'environ 0,2 mm.

9. Dispositif selon l'une des revendications 1 à 8,
**caractérisé en ce que** l'ouverture du profil est d'environ 0,4 mm à 0,6 mm.

10. Utilisation d'un dispositif selon l'une des revendications 1 à 9, dans laquelle l'écarteur (11, 12, 14) est inséré dans l'ouverture de pupille de l'iris (4) à travers un tunnel (5) pratiqué dans la sclérotique (9) ou dans la cornée de l'oeil et débouchant dans la chambre antérieure de l'oeil pour écarter l'iris de l'oeil.
